# EUROPÄISCHE PATENTSCHRIFT

(11) **EP 1 348 343 B1**
(45) Veröffentlichungstag und Bekanntmachung des Hinweises auf die Patenterteilung: **17.05.2006**
(21) Anmeldenummer: 03002929.2
(22) Anmeldetag: 10.02.2003
(51) Int. Cl.: A23L 1/30, A61K 36/00

(54) **Nahrungsergänzungsmittelpräparat**
Dietary supplement composition
Complément diététique

(30) Priorität: 28.03.2002 DE 10214297
(43) Veröffentlichungstag der Anmeldung: 01.10.2003
(73) Patentinhaber: PM-International AG, 1618 Luxembourg (LU)
(72) Erfinder: Schmitt, Gerhard, 64625 Bensheim (DE); Fritzmeyer, Franz-Paul Dr., 91710 Gunzenhausen (DE); Schwietz, Horst Dr., 90584 Allersberg (DE)
(74) Vertreter: Zech, Stefan Markus

(56) Entgegenhaltungen:
- US-A- 6 080 401
- INTERNET, [Online] XP002244928 Gefunden im Internet: <URL:http://www.roempp.com/thieme-chemistr y/roempp/prod/index.html> [gefunden am 2003-06-19]

## Beschreibung

Die Erfindung betrifft ein Nahrungsergänzungsmittelpräparat enthaltend probiotische Kulturen mit einem vorbestimmten Anteil an lebenden probiotischen Milchsäurebakterien sowie einen Gewürzextrakt nach dem ayurvedischen Prinzip - Maricha als Alkaloid. Ein derartiges Präparat ist in Form einer Arznei bereits aus der US 6 080 401 bekannt.

Es ist bereits bei einer Vielzahl von Nahrungsergänzungsmittelpräparaten, beispielsweise bei der Verabreichung von Beta-Karotin, Selen, Vitamin B6, Vitamin C, Vitamin A, Vitamin A + D festgestellt worden, dass die Beimengung eines Gewürzextraktes nach dem ayurvedischen Prinzip - Maricha eine resorptionsverstärkende Wirkung auslöst. Gewürzextrakt nach dem ayurvedischen Prinzip - Maricha scheint hier als eine Art Katalysator zu wirken, um die biologischen Barrieren, insbesondere Darmschleimhaut und Zellwände, besser zu durchdringen.

Die Aufgabe der vorliegenden Erfindung besteht gegenüber dem eingangs genannten Stand der Technik darin, ein Nahrungsergänzungsmittelpräparat bereitzustellen, welches diese vorteilhafte Wirkung von Gewürzextrakt nach dem ayurvedischen Prinzip - Maricha noch verstärkt.

Diese Aufgabe wird gelöst durch ein Nahrungsergänzungsmittelpräparat enthaltend neben einem Gewürzextrakt nach dem ayurvedischen Prinzip - Maricha als Alkaloid, probiotische Kulturen mit einem vorbestimmten Anteil an lebenden probiotischen Milchsäurebakterien, präbiotische Fruktooligosaccharide und/oder Inulin zur Förderung der probiotischen Kulturen sowie eine Mehrzahl verschiedener pflanzlicher Verdauungsenzyme.

Es hat sich gezeigt, dass die Wirkungsweise von Gewürzextrakt nach dem ayurvedischen Prinzip - Maricha noch unterstützt werden kann, wenn durch den Einsatz von probiotischen Kulturen eine insgesamt positive Darmflora und damit eine günstige Situation im Darmtrakt insbesondere an der Darmschleimhaut geschaffen werden kann. Die Darmschleimhaut wird durch die probiotischen Kulturen für besondere Wirkung des Gewürzextrakts nach dem ayurvedischen Prinzip - Maricha zweckmäßig vorbereitet. Um die probiotischen Kulturen in möglichst hoher Keimzahl an der Wirkungsstätte, d. h. im Darm, zu erhalten, sind dabei auch präbiotische Fruktooligosaccharide und/oder Inulin enthalten. Weiterhin ist eine Mehrzahl verschiedener pflanzlicher Verdauungsenzyme im erfindungsgemäßen Nahrungsergänzungsmittelpräparat enthalten.

In einer vorteilhaften Ausgestaltung enthält das Nahrungsergänzungsmittelpräparat weiterhin mindestens eine als Radikalfänger wirkende Substanz. Durch die in synergetischer Weise wirkenden probiotischen Kulturen einerseits und den Gewürzextrakt nach dem ayurvedischen Prinzip - Maricha andererseits wird der Radikalfänger zielgerichtet an die vorgesehenen Wirkungsorte transportiert.

In einer konkreten Ausgestaltung kann die mindestens eine als Radikalfänger wirkende Substanz Bioflavonoide umfassen.

Alternativ oder ergänzend kann die mindestens eine als Radikalfänger wirkende Substanz ein oder mehrere Antioxidantien umfassen.

Weiterhin ist es in zweckmäßiger Weise vorgesehen, dem Nahrungsergänzungsmittelpräparat eine Mehrzahl verschiedener löslicher und/oder unlöslicher Ballaststoffe beizumischen.

Die Resorption von Selen wird bekanntermaßen durch Gewürzextrakt nach dem ayurvedischen Prinzip - Maricha gesteigert. In einer vorteilhaften Ausgestaltung wird dem Nahrungsergänzungsmittelpräparat auch Selen beigemischt, wobei die erreichte Resorption durch die synergetische Wirkungsweise von proprobiotischen Kulturen einerseits und Gewürzextrakt nach dem ayurvedischen Prinzip - Maricha andererseits nochmals gesteigert ist.

In einer konkreten Ausgestaltung ist das Gewürzextrakt nach dem ayurvedischen Prinzip - Maricha in einer Konzentration enthalten, die einer Tagesdosis von 1 bis 10 mg, vorzugsweise 3 bis 7 mg, besonders bevorzugt etwa 5 mg pro Tag, entspricht.

Bevorzugtermaßen sind bezogen auf eine Tagesdosis von 5 mg in dem erfindungsgemäßen Präparat probiotische Kulturen mit einer Keimmenge von 10⁷ bis 10¹¹ Keimen, vorzugsweise 10⁸ bis 10¹⁰ Keimen, insbesondere von etwa 10⁹ Keimen enthalten. Die hier angegebenen Keimmengen beziehen sich auf die Anzahl lebender bzw. lebensfähiger Keime zum Zeitpunkt der Beimischung in das Präparat. Durch verschiedene, auch in der vorliegenden Anmeldung angegebene Maßnahmen lässt sich gewährleisten, dass ein möglichst hoher Anteil dieser Keime auch lebend an die Wirkstelle im Darmbereich gelangt.

In dem Nahrungsergänzungsmittelpräparat können weiterhin Zuschläge wie Träger- oder Geschmacksstoffe enthalten sein. In einer konkreten Ausgestaltung beläuft sich der Gewichtsanteil der Zuschläge am Präparat in einem Bereich zwischen 20 und 95 Gew.%, vorzugsweise im Bereich zwischen 60 und 95 Gew.%.

Das Präparat kann in flüssiger Form, in Form einer Emulsion oder in einer anderen beliebigen Form vorliegen. Bevorzugtermaßen ist das Präparat in pulvriger Form zur Herstellung einer wässrigen Lösung bereitgestellt.

## Patentansprüche

1. Nahrungsergänzungsmittelpräparat enthaltend:
- Probiotische Kulturen mit einem vorbestimmten Anteil an lebenden probiotischen Milchsäurebakterien,
- einen Gewürzextrakt nach dem ayurvedischen Prinzip - Maricha als Alkaloid,
- präbiotische Fruktooligosaccharide und/oder Inulin zur Förderung der probiotischen Kulturen und
- eine Mehrzahl verschiedener pflanzlicher Verdauungsenzyme.

2. Nahrungsergänzungsmittelpräparat nach Anspruch 1,
**dadurch gekennzeichnet,**
**dass** weiter mindestens eine als Radikalfänger wirkende Substanz enthalten ist.

3. Präparat nach Anspruch 2,
**dadurch gekennzeichnet,**
**dass** die mindestens eine als Radikalfänger wirkende Substanz Bioflavonoide umfasst.

4. Präparat nach Anspruch 2 oder 3,
**dadurch gekennzeichnet,**
**dass** die mindestens eine als Radikalfänger wirkende Substanz ein oder mehrere Antioxidantien umfasst.

5. Präparat nach einem der Ansprüche 1 bis 4,
**dadurch gekennzeichnet,**
**dass** weiterhin eine Mehrzahl verschiedener löslicher und/oder unlösliche Ballaststoffe enthalten ist.

6. Nahrungsergänzungsmittelpräparat nach einem der Ansprüche 1 bis 5,
**dadurch gekennzeichnet,**
**dass** das Spurenelement Selen enthalten ist.

7. Präparat nach einem der Ansprüche 1 bis 6,
**dadurch gekennzeichnet,**
**dass** das Gewürzextrakt nach dem ayurvedischen Prinzip - Maricha in einer Konzentration enthalten ist, die einer Tagesdosis von 1 bis 10 mg, vorzugsweise 3 bis 7 mg, besonders bevorzugt etwa 5 mg pro Tag entspricht.

8. Präparat nach einem der Ansprüche 1 bis 7,
**dadurch gekennzeichnet,**
**dass** bezogen auf eine Tagesdosis von 5 mg probiotische Kulturen mit einer Keimmenge von 10⁷ bis 10¹¹ Keimen, vorzugsweise 10⁸ bis 10¹⁰ Keimen insbesondere von etwa 10⁹ Keimen enthalten sind.

9. Präparat nach einem der Ansprüche 1 bis 8,
**dadurch gekennzeichnet,**
**dass** das Präparat weiterhin Zuschläge, wie Träger- oder Geschmacksstoffe enthält.

10. Präparat nach Anspruch 9,
**dadurch gekennzeichnet,**
**dass** der Gewichtsanteil der Zuschläge am Präparat im Bereich zwischen 20 und 95 Gewichtsprozent, vorzugsweise im Bereich zwischen 60 und 95 Gewichtsprozent liegt.

11. Präparat nach einem der Ansprüche 1 bis 10,
**dadurch gekennzeichnet,**
**dass** das Präparat in pulvriger Form zur Herstellung einer wässrigen Lösung vorliegt.

## Claims

1. Nutritional supplement preparation containing:
- probiotic cultures having a predetermined proportion of living probiotic lactic acid bacteria,
- a herbal extract according to the ayurvedic principle - maricha as alkaloid,
- prebiotic fructooligosaccharides and/or inulin for the promotion of the probiotic cultures and
- a plurality of different vegetable digestive enzymes.

2. Nutritional supplement preparation according to claim 1, **characterised in that** at least one substance acting as a free-radial absorber is also present.

3. Preparation according to claim 2, **characterised in that** the at least one substance acting as a free-radial absorber comprises bioflavonoids.

4. Preparation according to claim 2 or 3, **characterised in that** the at least one substance acting as a free-radial absorber comprises one or more anti-oxidants.

5. Preparation according to one of claims 1 to 4, **characterised in that** a plurality of different soluble and/or insoluble bulk materials are also present.

6. Nutritional supplement preparation according to one of claims 1 to 5, **characterised in that** the trace element selenium is present.

7. Preparation according to one of claims 1 to 6, **characterised in that** the herbal extract according to the ayurvedic principle - maricha is present in a concentration which corresponds to a daily dose of 1 to 10 mg, preferably 3 to 7 mg, particularly preferably about 5 mg per day.

8. Preparation according to one of claims 1 to 7, **characterised in that** probiotic cultures having a bacterial quantity of 10⁷ to 10¹¹ bacteria, preferably 10⁸ to 10¹⁰ bacteria, in particular of about 10⁹ bacteria, are present, based on a daily dose of 5 mg.

9. Preparation according to one of claims 1 to 8, **characterised in that** the preparation also contains additives, such as excipients or flavourings.

10. Preparation according to claim 9, **characterised in that** the weight proportion of the additives in the preparation lies in the range between 20 and 95 weight per cent, preferably in the range between 60 and 95 weight per cent.

11. Preparation according to one of claims 1 to 10, **characterised in that** the preparation exists in pulverulent form for the production of an aqueous solution.

## Revendications

1. Complément diététique, contenant
- des cultures probiotiques avec une proportion prédéfinie de lactobactéries probiotiques vivantes,
- un extrait d'épice d'après le principe ayurvédique - Maricha comme alcaloïde,
- des fructo-oligosaccharides pré-biotiques et/ou de l'inuline pour la promotion des cultures probiotiques et
- une pluralité de différentes enzymes digestives végétales.

2. Complément diététique selon la revendication 1,
**caractérisée en ce que**
en outre, au moins une substance agissant comme piège à radicaux est contenue.

3. Préparation selon la revendication 2,
**caractérisée en ce que**
l'au moins une substance agissant comme piège à radicaux comprend des bioflavonoïdes.

4. Préparation selon la revendication 2 ou 3,
**caractérisée en ce que**
l'au moins une substance agissant comme piège à radicaux comprend un ou plusieurs anti-oxydants.

5. Préparation selon l'une des revendications 1 à 4,
**caractérisée en ce que**
en outre, une pluralité de différents aliments encombrants solubles et/ou insoluble est contenue.

6. Préparation selon l'une des revendications 1 à 5,
**caractérisée en ce que**
l'oligo-élément sélénium est contenu.

7. Préparation selon l'une des revendications 1 à 6,
**caractérisée en ce que**
l'extrait d'épice d'après le principe ayurvédique - Maricha est contenu en une concentration qui correspond à une dose journalière de 1 à 10 mg, de préférence de 3 à 7 mg et de façon particulièrement préférée d'environ 5 mg par jour.

8. Préparation selon l'une dés revendications 1 à 7,
**caractérisée en ce que**,
rapporté à une dose journalière de 5 mg, des cultures probiotiques avec une quantité de germes de 10⁷ à 10¹¹ germes, de préférence de 10⁸ à 10¹⁰ germes, en particulier d'environ 10⁹ germes sont contenues.

9. Préparation selon l'une des revendications 1 à 8,
**caractérisée en ce que**
la préparation contient en outre des additifs tels des substances supports ou des aromates.

10. Préparation selon la revendication 9,
**caractérisée en ce que**
la proportion pondérale des additifs à la préparation est située dans l'intervalle entre 20 et 95 pour-cent en poids, de préférence dans l'intervalle entre 60 et 95 pour-cent en poids.

11. Préparation selon l'une des revendications 1 à 10,
**caractérisée en ce que**
la préparation se présente sous forme pulvérulente pour la fabrication d'une solution aqueuse.
